(19) Europäisches Patentamt
European Patent Office
Office européen des brevets

(11) Veröffentlichungsnummer : **0 298 031 B1**

# (12) EUROPÄISCHE PATENTSCHRIFT

(45) Veröffentlichungstag der Patentschrift : **10.04.91 Patentblatt 91/15**

(51) Int. Cl.$^5$ : **C07C 50/34,** C07D 303/20, C07C 59/90, C07C 243/28, C08L 63/00, C23C 18/16

(21) Anmeldenummer : **88810427.0**

(22) Anmeldetag : **22.06.88**

## (54) Substituierte Anthrachinone und härtbare Zusammensetzung.

(30) Priorität : **01.07.87 CH 2488/87**

(43) Veröffentlichungstag der Anmeldung : **04.01.89 Patentblatt 89/01**

(45) Bekanntmachung des Hinweises auf die Patenterteilung : **10.04.91 Patentblatt 91/15**

(84) Benannte Vertragsstaaten : **CH DE FR GB LI NL**

(56) Entgegenhaltungen :
**EP-A- 0 112 798**
**EP-B- 0 025 620**
**AT-B- 3 849**

(73) Patentinhaber : **CIBA-GEIGY AG**
**Klybeckstrasse 141**
**CH-4002 Basel (CH)**

(72) Erfinder : **Duthaler, Rudolf Dr.**
**Girenhaldenweg 17**
**CH-4126 Bettingen (CH)**
Erfinder : **Finter, Jürgen Dr.**
**Zasiusstrasse 100**
**W-7800 Freiburg (DE)**
Erfinder : **Ramanathan, Visvanathan Dr.**
**Friedensgasse 24**
**CH-4056 Basel (CH)**

EP 0 298 031 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegenden Erfindung betrifft Anthrachinone, die in 2-Stellung mit gegebenenfalls glycidylierten oder carboxyalkylierten Hydroxyphenylalkylgruppen substituiert sind ; eine härtbare Zusammensetzung, enthaltend a) ein Epoxidharz, b) gegebenenfalls einen Härter, c) diese Anthrachinone und d) einen Aminoalkohol, sowie die Verwendung der lichtempfindlichen gehärteten Zusammensetzung zur Herstellung von metallischen Ueberzügen oder Abbildungen durch stromlose Metallabscheidung.

In der EP-A-0 112 798 sind lichtempfindliche, vernetzte Reaktionsprodukte auf der Basis von Epoxidharzen beschrieben. Unter Mitverwendung von Metallsalzen der Gruppen Ib und VIII des Periodensystems der Elemente können durch Belichtung Metallkeime erzeugt werden, die man durch stromlose Metallabscheidung verstärken kann. Es ist wünschenswert, auf lichtempfindlichen Epoxidharzen direkt und ohne Mitverwendung von Metallsalzen metallische Ueberzüge oder Abbildungen durch stromlose Metallabscheidung zu erzeugen.

Ein Gegenstand vorliegender Erfindung sind Anthrachinon der Formel I

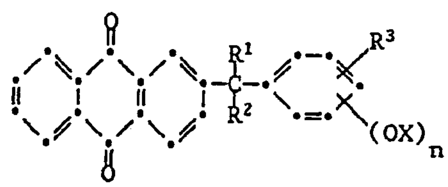 (I),

worin $R^1$ und $R^2$ unabhängig voneinander für H oder $C_1$-$C_5$-Alkyl stehen, $R^3$ H, $C_1$-$C_5$-Alkyl, $-CH_2C_6H_4OH$, oder $-C(CH_3)_2C_6H_4OH$ darstellt, n 1, 2 oder 3 bedeutet und X für H,

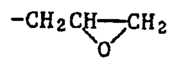

$-C_mH_{2m}COOH$ oder $-C_mH_{2m}CONR'NH_2$ steht, m eine Zahl von 1-12 ist und R' H oder $C_1$-$C_6$-Alkyl bedeutet.

$R^1$ und $R^2$ können verzweigtes und besonders lineares $C_1$-$C_5$-Alkyl sein. Als Alkyl stellen $R^1$ und $R^2$ bevorzugt Methyl oder Ethyl dar. Insbesondere sind $R^1$ und $R^2$ H.

$R^3$ stellt bevorzugt H oder lineares oder verzweigtes $C_1$-$C_5$-Alkyl dar. Als Alkyl ist $R^3$ bevorzugt Methyl oder Ethyl. Insbesondere ist $R^3$ H oder Methyl.

Der Rest X steht bevorzugt für H oder

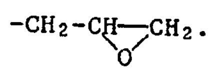

Bei der Gruppe $C_mH_{2m}$ kann es sich um verzweigtes und besonders lineares Alkylen handeln, wobei m bevorzugt eine Zahl von 1 bis 6 darstellt. Bei dem Alkylen handelt es sich bevorzugt um Methylen, Ethylen und Propylen. R' steht bevorzugt für H, Methyl oder Ethyl.

In einer bevorzugen Ausführungsform handelt es sich bei den Anthrachinonen der Formel I um solche worin $R^3$ H, X H oder

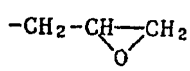

und n 1, 2 oder 3 bedeuten.

In einer anderen bevorzugten Ausführungsform handelt es sich bei den Anthrachinonen der Formel I um solche, worin $R^3$ Methyl, X H oder

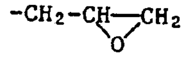

und n 1 sind.

Eine weitere bevorzugte Ausführungsform sind solche Anthrachinone der Formel I, worin $R^3$ H und n 1 oder 2 darstellen und die Gruppe OX in o-und/oder p-Stellung zur $-CR^1R^2$-Gruppe gebunden ist.

Eine andere bevorzuge Ausführungsform sind solche Anthrachinone der Formel I, worin $R^3$ $C_1$-$C_5$-Alkyl und n 1 sind, und $R^3$ in o-Stellung zur $-CR^1R^2$-Gruppe und $-OX$ in p-Stellung zu $R^3$ oder $-OX$ in o-Stellung zur $-CR^1R^2$-Gruppe und $R^3$ in p-Stellung zu $-OX$ gebunden sind.

Insbesondere handelt es sich bei den erfindungsgemässen Anthrachinonen um
2-[(2′-Hydroxyphen-1′-yl)methyl]anthrachinon,
2-[(4′-Hydroxyphen-1′-yl)methyl]anthrachinon,
2-[(2′,4′-Dihydroxyphen-1′-yl)methyl]anthrachinon,
2-[(2′,4′,6′-Trihydroxyphenyl)methyl]anthrachinon,
2-[(2′-Hydroxy-5-methylphen-1′-yl)methyl]anthrachinon,
2-[(2′-Methyl-5′-hydroxyphen-1′-yl)methyl]anthrachinon oder deren Glycidylderivate.

Ein weiterer Gegenstand der Erfindung ist ein Verfahren zur Herstellung von Anthrachinonen der Formel I gemäss Anspruch 1, dadurch gekennzeichnet, dass man ein 2-(Halogenmethyl)-anthrachinon der Formel II

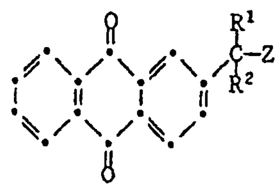

(II),

worin

$R^1$ und $R^2$ die zuvor angegebene Bedeutung haben und Z für Halogen steht, mit einem Phenol der Fomel III

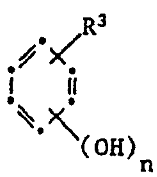

(III),

worin $R^3$ und n die zuvor angegebenen Bedeutungen haben, umsetzt und zur Herstellung von Verbindungen der Formel I mit

X = 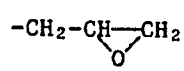

oder $-C_mH_{2m}COOH$ in Gegenwart eines HCl-Binders mit Epichlorhydrin bzw. $Z'$-$C_mH_{2m}COOR$, worin R der Rest eines Alkoholes ist und Z′ Halogen bedeutet, umsetzt und den Ester hydrolysiert oder mit $NHR'$-$NH_2$ umsetzt.

Die Verbindungen der Formeln II und III sind bekannt. Z in Formel II steht bevorzugt für Cl oder Br. Z′ ist vorzugsweise Cl oder Br. R als Rest eines einwertigen Alkohols stellt bevorzugt lineares oder verzweigtes $C_1$-$C_{12}$-Alkyl dar und ist besonders Methyl oder Ethyl.

Die Reaktion wird zweckmäsig bei Temperaturen von 50 bis 200°C, besonders 50 bis 150°C und Inertgas durchgeführt. Es können auch Lösungsmittel mitverwendet werden. Geeignet sind polare aprotische Lösungsmittel wie z.B. N-alkylierte Säureamide oder Lactame, Carbonsäureester und Lactone, Sulfone und Ether. Beispiele sind Dimethylformamid, Dimethylacetamid, N-Methylpyrrolidon, Dimethylsulfon, Tetramethylensulfon, Tetrahydrofuran und Dioxan.

Vorteilhaft wird die Reaktion mit Lewissäuren katalysiert, z.B. $ZnCl_2$, $FeCl_3$ der $SbF_3$. Die Verwendung von Lewissäuren ist besonders bei Monophenolen der Formel III (n = 1) zweckmässig.

Ueberraschend wurde gefunden, dass dann in Abwesenheit von Lewissäuren erfindungsgemässe Anthrachinone erhalten werden, wenn n in Formel III für 2 oder 3 steht. Diese Reaktion wird vorteilhaft in einem polaren aprotischen Lösungsmittel durchgeführt.

Die Glycidylisierung bzw. die Umsetzung mit $Z'C_mH_{2m}COOR$ und anschiessende Hydrolyse wird in bekannter Weise vorgenommen. Geeignete HCl-Binder sind z.B. Amine und besonders Alkalicarbonate wie z.B. $Na_2CO_3$ oder $K_2CO_3$.

Die Verbindungen der Formel I eignen sich zur Herstellung von lichtempfindlichen gehärteten Epoxidharzen. Ein weiterer Gegenstand der Erfindung ist eine härtbare Zusammensetzung, enthaltend

a) mindestens ein Epoxidharz mit durchschnittlich mehr als einer Epoxidgruppe im Molekül,

b) gegebenenfalls einen Härter für das Epoxidharz,

c) mindestens ein Anthrachinon der Formel I, und

d) mindestens ein primäres oder sekundäres aliphatisches Amin, das im aliphatischen Rest mindestens eine Hydroxylgruppe enthält.

Für die Verbindungen der Formel I gelten die zuvor angegebenen Bevorzugungen.

Das Epoxidharz enthält bevorzugt durchschnittlich mindestens 2 Epoxidgruppen im Molekül.

Als Epoxidharze kommen vor allem solche mit durchschnittlich mehr als einer an ein Heteroatom (z.B. Schwefel, vorzugsweise Sauerstoff oder Stickstoff) gebundenen Glycidylgruppe, β-Methylglycidylgruppe oder 2,3-Epoxycyclopentylgruppe in Frage ; genannt seien insbesondere Bis-(2,3-epoxycyclopentyl)-ether ; Di- bzw. Polyglycidylether von mehrwertigen aliphatischen Alkoholen, wie 1,4-Butandiol, oder Polyalkylenglykolen, wie Polypropylenglykole ; Di- oder Polyglycidylether von cycloaliphatischen Polyolen, wie 2,2-Bis-(4-hydroxycyclohexyl)-propan ; Di- bzw. Polyglycidylether von mehrwertigen Phenolen, wie Resorcin, Bis-(p-hydroxyphenyl)-methan, 2,2-Bis-(p-hydroxyphenyl)-propan (= Diomethan), 2,2-Bis-(4′-hydroxy-3′,5′-dibromphenyl)-propan, 1,1,2,2-Tetrakis-(p-hydroxyphenyl)-ethan, oder von unter sauren Bedingngen erhaltenen Kondensationsprodukten von Phenolen mit Formaldehyd wie Phenol-Novolake und Kresol-Novolake ; Di-bzw. Poly-(β-methylglycidyl)-ether der oben angeführten mehrwertigen Alkohole oder mehrwertigen Phenole ; Polyglycidylester von mehrwertigen Carbonsäuren, wie Phthalsäure, Terephthalsäure, $\Delta^4$-Tetrahydrophthalsäure und Hexahydrophthalsäure ; N-Glycidylderivate von Aminen, Amiden und heterocyclischen Stickstoffbasen, wie N,N-Diglycidylanilin, N,N-Diglycidyltoluidin, N,N,O-Triglycidyl-p-aminophenol, N,N,N′,N′-Tetraglycidyl-bis-(p-aminophenyl)-methan ; Triglycidyl-isocyanurat ; N,N′-Diglycidylethylenharnstoff ; N,N′-Diglycidyl-5,5-dimethylhydantoin, N,N′-Diglycidyl-5-isopropylhydantoin, N,N-Methylen-bis-(N′,N′-diglycidyl-5,5-dimethylhydantoin, 1,3-Bis-(N-glycidyl-5,5-dimethylhydantoin)-2-glycidyloxypropan ; N,N′-Diglycidyl-5,5-dimethyl-6-isopropyl-5,6-dihydro-uracil.

Eine bevorzugte Gruppe von Epoxidharzen sind glycidylierte Novolake®, Hydantoine, Aminophenole, Bisphenole oder aromatische Diamine. Besonders bevorzugte Zusammensetzungen enthalten als Epoxidharz einen glycidylierten Kresolnovolak, Bisphenol-A-diglycidylether, mit Bisphenol-A vorverlängerten Bisphenol-A-diglycidylether, Hydantoin-N,N′-bisglycid, Propylen-1,3-bis-hydantoin-2-hydroxy-triglycid, p-Aminophenoltriglycid, Diaminodiphenylmethan-tetraglycid oder Mischungen hiervon.

Geeignet sind auch vorreagierte Addukte solcher Epoxide mit Härtern für Epoxide, z.B. das erwähnte Addukt aus Bisphenol-A-diglycidylether und Bisphenol-A.

Als Härter für Epoxidharze kommen saure oder basische Verbindungen in Frage. Als geeignete Härter seien z.B. genannt : Amine, wie aliphatische, cycloaliphatische oder aromatische, primäre, sekundäre und tertiäre Amine, z.B. Ethylendiamin, Hexamethylendiamin, Trimethylhexamethylendiamin, Diethylentriamin, Triethylentetramin, Tetraethylenpentamin, N,N-Dimethylpropylen-1,3-diamin, N,N-Diethylpropylendiamin-1,3, 2,2-Bis-(4′-aminocyclohexyl)-propan, 3,5,5-Trimethyl-3-(aminomethyl)-cyclohexylamin ("Isophorondiamin"), Mannichbasen, wie 2,4,6-Tris-(dimethylaminomethyl)-phenol ; m-Phenylendiamin, p-Phenylendiamin, Bis-(4-aminophenyl)-methan, Bis-(4-aminophenyl)-sulfon, m-Xylylendiamin ; Addukte von Acrylnitril oder Monoepoxiden, wie z.B. Ethylenoxid oder Propylenoxid, an Polyalkylenpolyamine, wie z.B. Diethylentriamin oder Triethylentetramin ; Addukt aus Polyaminen, wie z.B. Diethylentriamin oder Triethylentetramin, im Ueberschuss und Polyepoxiden, wie z.B. Diomethanpolyglycidylethern ; Addukte aus Monophenolen oder Polyphenolen und Polyamiden ; Polyamide, insbesondere solche aus aliphatischen Polyaminen, wie z.B. Diethylentriamin oder Triethylentetramin, und di- oder trimerisierten ungesättigten Fettsäuren, wie z.B. dimerisierte Leinölfettsäure (VERSAMID®) ; Polysulfide (THIOKOL®) ; Anilin-Formaldehyde ; mehrwertige Phenole, z.B. Resorcin, 2,2-Bis-(4-hydroxyphenyl)-propan oder Phenol-Formaldehyd-Harze ; mehrbasische Carbonsäuren und ihre Anhydride, z.B. Phthalsäureanhydrid, $\Delta^4$-Tetrahydrophthalsäureanhydrid, Hexahydrophthalsäureanhydrid, 4-Methylhexahydrophthalsäureanhydrid, 3,6-Endomethylen-$\Delta^4$-tetrahydrophthalsäureanhydrid, 4-Methyl-3,6-endomethylen-$\Delta^4$-tetrahydrophthalsäureanhydrid (= Methylnadicanhydrid), 3,4,5,6,7-Hexachlor-3,6-endomethylen-$\Delta^4$-tetrahydrophthalsäureanhydrid, Bernsteinsäureanhydrid, Adipinsäureanhydrid, Trimethyladipinsäureanhydrid, Azelainsäureanhydrid, Sebacinsäureanhydrid, Maleinsäureanhydrid, Dodecylbernsteinsäureanhydrid ; Pyromellitsäuredianhydrid, Trimellitsäureanhydrid, Benzophenontetracarbonsäuredianhydrid oder Gemische solcher Anhydride.

Eine bevorzugte Gruppe von Härtern sind Novolake®, Polyaminoamide und Carbonsäureanhydride.

Die erfindungsgemässe Zusammensetzung kann auch Härtungsbeschleuniger bzw. Polymerisationsinitiatoren oder thermische bzw. photochemische Härtungskatalysatoren enthalten. Beispiele sind : tertiäre Amine, deren Salze oder quaternäre Ammoniumverbindungen, z.B. 2,4,6-Tris-(dimethylaminomethyl)phenol, Benzyldimethylamin, 2-Aethyl-4-methylimidazol, Triamylammoniumphenolat ; Mono- oder Polyphenole wie Phenol oder Diomethan oder Salicylsäure ; Dicyandiamid ; Bortrifluorid und sein Komplexe mit organischen Verbindungen, wie $BF_3$-Ether-Komplexe und $BF_3$-Amin-Komplexe, z.B. $BF_3$-Monoethylamin-Komplex ; Acetoacetanilid-$BF_3$-Komplex ; Phosphorsäure ; Triphenylphosphit. Als photoempfindliche Initiatoren eignen sich z.B. Oniumsalze oder Metallkomplexesalze wie z.B. Diazoniumsalze von aromatischen Aminen, Triphenylsulfonium- oder Diphenyliodoniumsalze oder Cyclopentadienyleisenarensalze.

Härtungsbeschleuniger und -katalysatoren werden üblicherweise in einer Menge von 0,1-10 Gew.-% zugegeben, bezogen auf das Epoxidharz. Härter für Epoxidharze werden im allgemeinen in äquimolaren Mengen verwendet, bezogen auf die Epoxidgruppen und funktionellen Gruppen eines Härters.

Die Zusammensetzung enthält das Anthrachinon der Formel I bevorzugt in einer Menge von 0,1-1, besonders 0,2-0,8 Mol/kg Epoxidharz, und das Hydroxylgruppen enthaltende Amin bevorzugt in einer Menge von 0,1-1,2, besonders 0,3-1 Mol/kg Epoxidharz. Zusätzliche Härter sind bevorzugt in einer Menge von 0,1-0,5, besonders 0,1-0,3 Mol/kg Epoxidharz enthalten.

Wenn in Formel I n = 1 ist, verwendet man vorteilhaft ein Epoxidharz mit durchschnittlich mindestens 3 Epoxidgruppen im Molekül oder zusätzlich einen Härter.

Bei den Aminen der Komponente d) kann es sich um Hydroxylgruppen enthaltende aliphatische Amine mit 2 bis 30, vorzugsweise 2 bis 20 C-Atomen mit 1 bis 3, vorzugsweise 1 Hydroxylgruppen im aliphatischen Rest handeln. Der aliphatische Rest kann linear oder verzweigt sein und durch –O– oder Aminogruppen unterbrochen sein. Bevorzugt enthält der aliphatische Rest primäre OH-Gruppen. In einer bevorzugten Ausführungsform entspricht das Amin der Formel IV

$$H\!-\!\overset{R^4}{\overset{|}{N}}\!-\!C_yH_{2y+1-x}(OH)_x \qquad (IV),$$

worin $R^4$ H, lineares oder verzweigtes $C_1$-$C_{18}$-Alkyl, $C_3$-$C_7$-Cycloalkyl, $C_6$-$C_{10}$-Aryl, $C_7$-$C_{18}$-Alkaryl, $C_7$-$C_{12}$-Aralkyl, $C_8$-$C_{18}$-Alkaralkyl oder die Gruppe $-C_yH_{2y+1-x}OH_x$ bedeutet, x für eine Zahl von 1 bis 3 und y für eine Zahl von 2 bis 12 stehen, wobei die Gruppe $C_yH_{2y}$ durch ein oder mehrere –O– oder $-NR^4-$ unterbrochen sein kann.

$R^4$ enthält als Alkyl bevorzugt 1 bis 12 und besonders 1 bis 6 C-Atome. $R^4$ ist als Alkyl bevorzugt Methyl oder Ethyl. Insbesondere stellt $R^4$ H dar. Als Cycloalkyl enthält $R^4$ vorzugsweise 5 oder 6 Ring-C-Atome und ist z.B. Cyclopentyl oder Cyclohexyl.

$R^4$ kann in der Bedeutung von Aryl z.B. Naphthyl und besonders Phenyl sein. $R^4$ in der Bedeutung von Alkaryl ist besonders $C_7$-$C_{18}$-Alkylphenyl, zum Beispiel Methyl-, Ethyl-, Dimethyl-, n- und i-Propyl-, n-, i- und t-Butyl-, Pentyl-, Hexyl-, Octyl-, Nonyl-, Decyl- und Dodecylphenyl. Bei $R^4$ in der Bedeutung von Aralkyl kann es sichum 1-oder 2-Phenyleth-1-yl oder besonders Benzyl handeln. Wenn $R^4$ Alkaralkyl ist, handelt es sich bevorzugt um Alkylbenzyl mit insbesondere 8 bis 14 C-Atomen, z.B. Methyl-, Ethyl-, Dimethyl-, n-und i-Propyl-, n-, i- und t-Butyl-, Pentylund Hexylbenzyl. Sofern die Gruppe $C_yH_{2y}$ durch –O– unterbrochen ist, kann es sich um Oxaalkylenreste handeln, die z.B. der Formel $-R^5\text{-}(OR^6)_t-$ entsprechen können, worin $R^5$ und $R^6$ unabhängig voneinander lineares oder verzweigtes $C_2$-$C_6$-Alkylen und t eine Zahl von 2 bis 6 sind. Sofern die Gruppe $C_yH_{2y}$ durch $-NR^4-$ unterbrochen ist, kann die Gruppe vorzugsweise der Formel

$$-\!\left(\!-R^7-NH-\right)_s\!-R^8- ,$$

entsprechen, worin $R^7$ lineares oder verzweigtes $C_2$-$C_6$-Alkylen, bevorzugt Ethylen, $R^8$ lineares oder verzweigtes $C_1$-$C_{10}$-, vorzugsweise $C_2$-$C_6$-Alkylen und s eine Zahl von 1 bis 3 sind. In Formel IV steht y besonders für eine Zahl von 2 bis 7.

Eine bevorzugte Untergruppe sind solche Amine der Formel IV, worin $R^4$ für H steht, y eine Zahl von 2 bis 7 und x eine Zahl von 1 bis 3 bedeuten.

Beispiele für Amine der Formel IV sind : Ethanolamin, 1-Amino-2-hydroxypropan, 1-Amino-3-hydroxypropan, 1-Amino-4-hydroxybutan, 1-Amino-5-hydroxypentan, 1-Amino-6-hydroxyhexan, Aminotrimethylolmethan, Aminodimethylolmethan, Aminomethyldimethylolmethan, Aminomethyltrimethylolmethan, Hydroxyethoxyethylamin, Hydroxypropoxyethylamin, N-(Hydroxyethyl)ethylendiamin, N-(Hydroxyethyl)-diethylentriamin,

$$H_2N(CH_2CH_2O-)_{2-6}H.$$

Primäre aliphatische Hydroxylgruppen enthaltende Amine sind Härter für Epoxidharze, wobei lineare Polymere erhalten werden, wenn Epoxidharze mit 2 Epoxidgruppen im Molekül verwendet werden. Bei Mitverwendung anderer Härter werden vernetzte Polymere erhalten.

Bei Verwendung der monofunktionellen Anthrachinone der Formel I werden vorteilhaft Epoxidharze mit mindestens 3 Epoxidgruppen im Molekül verwendet, z.B. epoxidierte Novolake, um vernetzte Epoxidharze zu erhalten.

Die erfindungsgemässen Zusammensetzungen sind härtbar, wobei die gehärteten bzw. vernetzten Epoxidharze lichtempfindlich sind. Auf den belichteten Stellen der Oberfläche können durch stromlose Metallabscheidung dünne Schichten von Metallen wie z.B. Kupfer abgeschieden werden.

Die Härtung der Zusammensetzung erfolgt in bekannter Weise, wobei vor oder mit der Härtung eine Formgebung nach den üblichen Formgebungsverfahren möglich ist, z.B. die Herstellung von Beschichtungen auf einem Trägermaterial durch Spritzen, Streichen oder Rakeln, oder die Herstellung von Formteilen mittels Giesstechniken, oder die Herstellung von Verbundkörpern mittels Tränk- und Pressverfahren.

Das Hydroxylgruppen enthaltende Amin der Komponente d) kann in einer bevorzugten Ausführungsform mit dem Epoxidharz der Komponente a) zu Addukten vorreagiert und dann mit einem als Härter wirkenden Anthrachinon der Formel I gegebenenfalls zusammen mit Härtern für Epoxidharze vermischt und vernetzt werden. Geeignete Härter von Anthrachinonen der Formel I sind insbesondere solche, worin X H und n 2 oder 3 ist.

Die Glycidylderivate der Formel I sind selbst Epoxidharze. Zur Herstellung von vernetzten Produkten wird ein Härter, z.B. ein Novolak® mitverwendet.

Bei Verwendung von Anthrachinonen der Formel I, worin n = 1 ist, kann es vorteilhaft sein, einen Härter für Epoxidharze mitzuverwenden, besonders einen Novolak-, Amin- oder Anhydridhärter. Zweckmässig wird das monofunktionelle Anthrachinon zusammen mit dem –OH enthaltenden Amin und dem Epoxid vorreagiert und darauf mit einem Härter vermischt und ausgehärtet.

Neben einer stufenweisen Härtung ist auch das Vermischen aller Komponenten und die darauffolgende Härtung möglich.

Das Vermischen der Komponenten erfolgt nach üblichen Verarbeitungsmethoden gegebenenfalls zusammen mit einem Lösungsmittel. Es können weitere für die Verarbeitung oder die Verbesserung der Eigenschaften der gehärteten Epoxidharze übliche Zusätze zugegeben werden, z.B. Weichmacher, Farbstoffe, Pigmente, Füllstoffe, Formtrennungsmittel oder H-Donoren. Für die Abscheidung von Metallen kann ein Gehalt an Metallsalzen oder Metallkomplexen der Gruppen Ib oder VIII des periodischen Systems der Elemente vorteilhaft sein, z.B. in einer Menge von 0,01 bis 10 Gew.-%, bezogen auf die Zusammensetzung.

Die Härtung wird im allgemeinen bei Temperaturen von 20 bis 200°C, besonders 50 bis 150°C vorgenommen.

Die gehärteten Zusammensetzungen sind ein weiterer Gegenstand der Erfindung.

Die gehärteten Zusammensetzungen sind lichtempfindlich. Die belichteten Teile erscheinen dunkler als die unbelichteten Teile. Auf den belichteten Teilen können mit konventionellen Metallabscheidungsbädern (siehe z.B. US-PS 4 510 279), besonders solchen mit z.B. Nickel- oder Kupfersalzen, direkt Metalle abgeschieden werden. So können z.B. gedruckte Schaltungen hergestellt werden. Die belichteten Epoxidharze können auch für optische Speicherungen verwendet werden.

Ein weiterer Gegenstand der Erfindung ist die Verwendung einer gehärteten erfindungsgemässen Zusammensetzung zur Herstellung von metallischen Ueberzügen oder Abbildungen durch stromlose Metallabscheidung nach einer vollständigen oder teilweisen Belichtung der Oberfläche.

Die Belichtung wird bevorzugt mit UV-Licht vorgenommen. Es können beliebige Lichtquellen eingesetzt werden, wobei die Verwendung von UV-Lampen bevorzugt ist. Geeignete Lichtquellen sind z.B. Xenonlampen, Metallhalogenidlampen und besonders Quecksilberhoch- und -mitteldrucklampen.

Zur Herstellung der metallischen Ueberzüge oder Abbildungen kann man so vorgehen, dass man die erfindungsgemäss, sich gegebenenfalls als Schicht auf einem Trägermaterial befindliche erfindungsgemässe Zusammensetzung härtet, darauf flächenmässig oder durch eine Bildvorlage belichtet und danach mit einem Metallabscheidungsbad behandelt.

Die Mitverwendung eines Metallsalzes oder Metallkomplexes ist überflüssig. Die abgeschiedenen Metalle haften fest auf der Epoxidharzoberfläche ; eine Vorbehandlung ist nicht notwendig. Ferner weisen die gehärteten Zusammensetzung erhöhte Glasumwandlungstemperaturen auf.

Die nachfolgenden Beispiele erläutern die Erfindung näher.

A) Herstellungsbeispiele :

Beispiel 1 :

1,5 g 2-(Brommethyl)anthrachinon und 6,3 g Phloroglucin werden in 20 ml Dimethylformamid unter Rühren 1 Stunde lang unter Rückfluss erhitzt. Dann wird ca. die Hälfte Dimethylformamid abdestilliert und der Rest mit Wasser verdünnt. Das ausgefallene Produkt wird abfiltriert, mit Wasser gewaschen und getrocknet. Durch Umkristallisieren aus Essigester wird 1,3 g 2-[(2′,4′,6′-Trihydroxyphen-1-yl)methyl]anthrachinon erhalten. Schmelzpunkt (Smp.) > 240°C. Die Struktur wird durch 1H-NMR- und Massenspektren bestätigt.

Beispiel 2 :

2,6 g 2-(Chlormethyl)anthrachinon, 8,8 g Resorcin und 1 g Kaliumiodid werden in 40 ml Dimethylformamid unter Inertgas und Rühren während 18 Stunden unter Rückfluss erhitzt. Dann wird ca. die Hälfte Dimethylformamid abdestilliert und der Rest mit Wasser verdünnt. Das ausgefallene Produkt wird abfiltriert, mit Wasser gewaschen und getrocknet. Durch Umkristallisieren aus Ethanol wird 2-[(2′,4′-Dihydroxyphen-1-yl)methyl]anthrachinon erhalten. Smp. : 227-228°C. Die Struktur wird durch $^1$H-NMR- und Massenspektren bestätigt.

Beispiel 3 :

1 g 2-(Chlormethyl)anthrachinon, 4 g Phenol und 0,2 g Zinkchlorid werden unter Rühren während 3 Stunden bei 100°C erhitzt. Das überschüssige Phenol wird durch Wasserdampfdestillation entfernt. Der gelbe Rückstand wird in Essigester aufgenommen und mit Magnesiumsulfat getrocknet. Das Produkt wird durch Chromatographie an einer Kieselgelsäule mit Methylenchlorid, Hexan und Essigester (5 : 5 : 1) als Eluiermittel in zwei Fraktionen aufgetrennt. Die erste Fraktion ist 2-[(2′-Hydroxyphen-1-yl)methyl]anthrachinon, Smp. : 190-191°C. Elementaranalyse : berechnet C 80,24, H 4,49, O 15,27% ; gefunden C 79,6, H 4,7, O 15,1%. Die zweite Fraktion ist 2-[(4′-Hydroxyphen-1-yl)methyl]anthrachinon, Smp. : 189-190°C. Elementaranalyse : berechnet C 80,24, H 4,49, O 15,27% ; gefunden C 79,6, H 4,7, O 15,5%. Die Strukturen der zwei Verbindungen werden durch $^1$H-NMR- und Massenspektren bestimmt.

Beispiel 4 :

1 g 2-(Chlormethyl)anthrachinon, 4 g p-Kresol und 0,2 g Zinkchlorid werden unter Rühren während 12 Stunden bei 100°C erhitzt. Das überschüssige Kresol wird durch Wasserdampfdestillation entfernt. Der gelbe Rückstand wird in Essigester gelöst und mit Magnesiumsulfat getrocknet. Das Produkt wird durch Chromatographie an einer Kieselgelsäule mit Methylenechorid, Hexan und Essigester (5 : 5 : 1) als Eluiermittel in zwei Fraktionen aufgetrennt. Die erste Fraktion ist 2-[(2′-Hydroxy-5′-methylphen-1-yl)methyl]anthrachinon, Smp. : 174-176°C. Elementaranalyse : berechnet C 80,47, H 4,91, O 14,62% ; gefunden C 80,4, H 4,9, O 14,6%. Die zweite Fraktion ist 2-[(5′-Hydroxy-2′-methylphen-1-yl)methyl]-anthrachinon, Smp. : 191-192°C. Elementaranalyse : berechnet C 80,47, H 4,91, O 14,62% ; gefunden C 80,5, H 5,0, O 14,1%. Die Strukturen der zwei Verbindungen werden an Hand von $^1$H-NMR- und Massenspektren zugeteilt.

Beispiel 5 :

9,9 g 2-[(2′,4′-Dihydroxyphen-1-yl)methyl]anthrachinon, 11 g Epichlorhydrin und 18 g wasserfreies Kaliumkarbonat werden in 200 ml Ethylmethylketon unter Inertgas und Rühren während 24 Stunden unter Rückfluss erhitzt. Die Suspension wird abfiltriert und das Filtrat mit Ethylmethylketon gewaschen. Die gesamte Ethylmethylketonlösung wird eingedampft und das Produkt wird durch Flashchromatographie an einer Kieselgelsäule mit Methylenchlorid, Toluol und Diethylether (13 : 6 : 1) als Eluiermittel gereinigt. Man erhält 2-[(2′,4′-Diglycidyloxyphen-1-yl)methyl]anthrachinon, Smp. : 161-162°C. Elementaranalyse : berechnet C 73,29, H 5,01, O 21,70% ; gefunden C 73,2, H 4,9, O 21,6%. Die Struktur wird durch $^1$H-NMR- und Massenspektren bestätigt.

Beispiel 6 :

1,7 g 2-[(2′,4′,6′-Trihydroxyphen-1-yl)methyl]anthrachinon, 2,4 g Epichlorhydrin und 4,8 g wasserfreies Kaliumkarbonat werden in 20 ml Dimethylformamid unter Inertgas und Rühren während 12 Stunden bei 70-75°C erhitzt. Nach beendeter Reaktion wird das Gemisch mit Wasser verdünnt und mit Essigester extrahiert.

Das Essigesterextrakt wird mit Wasser gewaschen, über Magnesiumsulfat getrocknet und dann eingedampft. Das Produkt wird durch Flashchromatographie an einer Kieselgelsäule mit Methylenchlorid, Hexan und Essigester (5 : 5 : 1) als Eluiermittel gereinigt. Man erhält 2-[(2',4',6'-Triglycidyloxyphen-1-yl)methylanthrachinon, Smp.: 177-178°C. Elementaranalyse : berechnet C 70,03, H 5,09, O 24,87% ; gefunden C 70,1, H 5,2, O 24,7%. Die Struktur wird durch $^1$H-NMR- und Massenspektren bestätigt.

B) Anwendungsbeispiele :

Beispiele 7-10 :

2,382 g (0,007 mol) Bisphenol-A-diglycidylether wird in 5 ml N-Methylpyrrollidon bei 40°C gelöst. Zu dieser Lösung gibt man 0,225 g (0,003 mol) Propanolamin unter Rühren zu, steigert die Temperatur auf 120°C und gibt nach 3h Rühren 0,991 g (0,003 mol) substituiertes Anthrachinon zu. Es wird eine rote Lösung erhalten, die man bei gleicher Temperatur für 3 h ausreagieren lässt. Das Molekulargewicht, bestimmt durch GPC (geeicht mit Addukten aus Bisphenol A und Bisphenol-A-diglycylether) beträgt 670 Dalton. Man lässt abkühlen und versetzt mit 0,246 g eines Kresolnovolaks® mit einem Hydroxyäquivalentgewicht von 123,15 g Mol Hydroxid und lässt bei Raumtemperatur rühren. Die Lösung wird als Film mittels Drahtrakeln auf Aluminium oder Polyesterträger gegossen und im Umluftofen für 12 h bei 80°C getrocknet, und dann bei 140°C für 4 h ausgehärtet.

Tabelle 1 zeigt die Zusammensetzung und die Eigenschaften der Polyaddukte.

Die Effizienz der Protoreduktion wird wie folgt bestimmt. Ein Film auf einem Polyesterträger mit O.D. = 1 bei 324 nm wird mit einer Hg-Hochdrucklampe mit 40 mW/cm$^{-2}$ bestrahlt und in regelmässigen Abständen das UV/VIS - Spektrum aufgenommen. Die Bande bei 324 nm nimmt ab, die Bande bei 386 nm nimmt zu. Das Verhältnis beider Banden nach 2 Minuten Belichtungszeit wird als Effizienz genommen.

Photometallisierung

Filme auf einem Polyesterträger werden auf einem thermostatisierbaren Vakuumheiztisch bei 50°C durch ein Negativ mit einer HG-Hochdrucklampe mit 40 mW/cm$^{-2}$ Intensität belichtet.

Man erhält ein dunkles,negatives Abbild der Vorlage, das in einem Abscheidungsbad der Zusammensetzung,

| | |
|---|---|
| $CuSO_4 \times 5H20$ | 0,0665 Mol/l |
| HCOH | 0,0467 Mol/l |
| Quadrol | 0,0599 Mol/l |
| NaOH | pH 12,6 |
| NaCH | 25 ml/l |
| 2-Mercaptobenzthiazol | 10 ml/l |

bei 45°C zu einem metallischen Kupferbild verstärkt wird.

Tabelle 1

| Bsp. No. | Anthrachinon (AQ) gemäss Beispiel | Zusammensetzung (mMol) | | | | Tg °C | O.D.(386 nm) / O.D.(324 nm) |
|---|---|---|---|---|---|---|---|
| | | AQ | Aminoethanol | Bisphenol-A-diglycidylether | Novolak® (mAequ.) | | |
| 7 | 1 | 3 | 3 | 7 | 2 | 121 | 0,55 |
| 8 | 2 | 3 | 3 | 7 | 2 | 76 | 0,57 |
| 9 | 5 | 3 | 3 | 4[1]) | 2 | 101,5 | 0,64 |
| 10 | 6 | 3 | 3 | 4[1]) | 2 | 113 | 0,68 |

[1]) Mit 3 mMol Bisphenol-A vorverlängert

Beispiele 11-13 :

Es wird wie in den Beispielen 7-10 verfahren und zusätzlich 6 mMol 5,5'-Dimethyl-N,N'-hydantoindiglycidyl mitverwendet. Die Zusammensetzungen und Ergebnisse sind in Tabelle 2 zusammengefasst.

Tabelle 2

| Bsp. No. | Anthrachinon (AQ) gemäss Beispiel | Zusammensetzung (mMol) | | | | Tg °C | O.D.(386 nm) / O.D.(324 nm) |
|---|---|---|---|---|---|---|---|
| | | AQ | 2-Propanolamin | Epoxid | Novolak | | |
| 11 | 2 | 3 | 3 | 1[a)] | 1 | 87 | 0,55 |
| 12 | 5 | 3 | 4 | 1[b)] | 6 | 92 | 0,55 |
| 13 | 2 | 3 | 3 | 1[c)] | 1 | 94 | 0,50 |

a) mit Bisphenol-A vorverlängerter Bisphenol-A-diglycidylether (Epoxyäquivalentgewicht 901 g/mol)

b) Bisphenol-A-diglycidylether

c) epoxidierter Kresolnovolak® (Epoxyäquivalentgewicht 223,7 g/mol)

## Ansprüche

1. Anthrachinone der Formel I

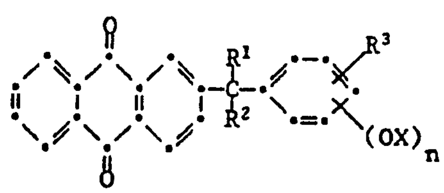

(I),

worin $R^1$ und $R^2$ unabhängig voneinander für H oder $C_1$-$C_5$-Alkyl stehen, $R^3$ H, $C_1$-$C_5$-Alkyl, $-CH_2C_6H_4OH$, oder $-C(CH_3)2C_6H_4OH$ darstellt, n 1, 2 oder 3 bedeutet und X für H,

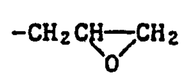

oder $-C_mH_{2m}COOH$ oder $-C_mH_{2m}CONR'NH_2$ steht, m eine Zahl von 1-12 ist und $R^1$ H oder $C_1$-$C_6$-Alkyl bedeutet.

2. Anthrachinone der Formel I gemäss Anspruch 1, worin $R^1$ und $R^2$ als Alkyl unabhängig voneinander Methyl oder Ethyl sind, oder $R^1$ und $R^2$ H bedeuten.

3. Anthrachinone der Formel I gemäss Anspruch 1, worin $R^3$ H oder Methyl ist.

4. Anthrachinone der Formel I gemäss Anspruch 1, worin $R^3$ H, X H oder

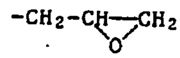

und n 1, 2 oder 3 bedeuten.

5. Anthrachinone der Formel I gemäss Anspruch 1, worin $R^3$ Methyl, X H oder

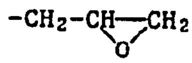

und n 1 sind.

6. Anthrachinone der Formel I gemäss Anspruch 1, worin $R^3$ H und n 1 oder 2 darstellen und die Gruppe OX in o- und/oder p-Stellung zur $-CR^1R^2$-Gruppe gebunden ist.

7. Anthrachinone der Formel I gemäss Anspruch 1, worin $R^3$ $C_1$-$C_5$-Alkyl und n 1 sind, und $R^3$ in o-Stellung zur $-CR^1R^2$-Gruppe und $-OX$ in p-Stellung zu $R^3$ oder $-OX$ in o-Stellung zur $-CR^1R^2$-Gruppe und $R^3$ in p-Stellung zu -OX gebunden sind.

8. Anthrachinone der Formel I gemäss Anspruch 1, dadurch gekennzeichnet, dass es sich um

2-[(2′-Hydroxyphen-1′-yl)methyl]anthrachinon,
2-[(4′-Hydroxyphen-1′-yl)methyl]anthrachinon,
2-[(2′,4′-Dihydroxyphen-1′-yl)methyl]anthrachinon,
2-[(2′,4′,6′-Trihydroxyphenyl)methyl]anthrachinon,
2-[(2′-Hydroxy-5-methylphen-1′-yl)methyl]anthrachinon,
2-[(2′-Methyl-5′-hydroxyphen-1′-yl)methyl]anthrachinon oder deren Glycidylderivate handelt.

9. Verfahren zur Herstellung von Anthrachinonen der Formel I gemäss Anspruch 1, dadurch gekennzeichnet, dass man ein 2-(Halogenmethyl)-anthrachinon der Formel II

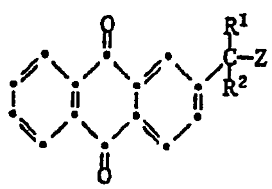

(II),

worin

$R^1$ und $R^2$ die zuvor angegebene Bedeutung haben und Z für Halogen steht, mit einem Phenol der Fomel III

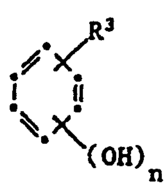

(III),

worin $R^3$ und n die zuvor angegebenen Bedeutungen haben, umsetzt und zur Herstellung von Verbindungen der Formel I mit

X =

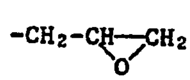

oder $-C_mH_{2m}COOH$ in Gegenwart eines HCl-Binders mit Epichlorhydrin bzw. $Z'$-$C_mH_{2m}COOR$, worin R der Rest eines Alkoholes ist, umsetzt und den Ester hydrolysiert oder mit $NHR'NH_2$ umsetzt.

10. Verfahren gemäss Anspruch 9, dadurch gekennzeichnet, dass man die Reaktion ohne Lewiskatalysator durchführt, wenn in Formel III n 2 oder 3 ist.

11. Verfahren gemäss Anspruch 10, dadurch gekennzeichnet, dass es in einem polaren aprotischen Lösungsmittel durchgeführt wird.

12. Härtbare Zusammensetzung, enthaltend

a) mindestens ein Epoxidharz mit durchschnittlich mehr als einer Epoxidgruppe im Molekül,

b) gegebenenfalls einen Härter für das Epoxidharz,

c) mindestens ein Anthrachinon der Formel I gemäss Anspruch 1, und

d) mindestens ein primäres oder sekundäres aliphatisches Amin, das im aliphatischen Rest mindestens eine Hydroxylgruppe enthält.

13. Zusammensetzung gemäss Anspruch 12, worin

c) das Anthrachinon der Formel I in einer Menge von 0,1-1 Mol/kg Epoxidharz, und

d) das Amin in einer Menge von 0,1-1,2 Mol/kg Epoxidharz enthalten sind.

14. Zusammensetzung gemäss Anspruch 12, worin als Epoxidharz glycidylierte Novolake®, Hydantoine, Aminophenole, Bisphenole oder aromatische Diamine enthalten ist.

15. Zusammensetzung gemäss Anspruch 14, worin das Epoxidharz ein epoxidierter Kresolnovolak, Bisphenol-A-diglycidylether, mit Bisphenol-A vorverlängerter Bisphenol-A-diglycidylether, 5.5-Dimethylhydantoin-N,N'-bisglycid, 1,3-Bis(N',N'-diglycidyl-5,5-dimethylhydanto-4-yl)-2-glycidoxypropan, p-Aminophenoltriglycid, Diaminodiphenylmethan oder Mischungen hiervon ist.

16. Zusammensetzung gemäss Anspruch 12, worin es sich bei dem Härter b) um einen Novolak, ein Polyaminoamid oder ein Polycarbonsäureanhydrid handelt.

17. Zusammensetzung gemäss Ansprüche 12, worin das Amin der Komponente d) der Formel IV

$$H-\overset{R^4}{N}-C_yH_{2y+1-x}(OH)_x \qquad (IV)$$

entspricht, worin $R^4$ H, lineares oder verzweigtes $C_1$-$C_{18}$-Alkyl, $C_3$-$C_7$-Cycloalkyl, $C_6$-$C_{10}$-Aryl, $C_7$-$C_{18}$-Alkaryl, $C_7$-$C_{17}$-Aralkyl, $C_8$-$C_{18}$-Alkaralkyl oder die Gruppe $-C_yH_{2y+1-x}(OH)_x$ bedeutet, x für eine Zahl von 1 bis 3 und y für eine Zahl von 2 bis 12 stehen, wobei die Gruppe $C_yH_{2y}$ durch ein oder mehrere –O– oder $-NR^4$ unterbrochen sein kann.

18. Zusammensetzung gemäss Anspruch 12, worin in Formel IV $R^4$ für H steht, y eine Zahl von 2 bis 7 und x eine Zahl von 1 bis 3 bedeuten.

19. Gehärtete Zusammensetzung aus

a) mindestens einem Epoxidharz mit durchschnittlich mehr als einer Epoxidgruppe im Molekül,

b) gegebenenfalls einem Härter für Epoxidharz,

c) mindestens einem Anthrachinon der Formel I gemäss Anspruch 1, und

d) mindestens einem primären oder sekundären aliphatischen Amin, das im aliphatischen Rest mindestens eine Hydroxylgruppe enthält.

20. Verwendung einer gehärteten Zusammensetzung gemäss Anspruch 19 zur Herstellung von metallischen Ueberzügen oder Abbildungen durch stromlose Metallabscheidung nach einer vollständigen oder teilweisen Belichtung der Oberfläche.

## Claims

1. An anthraquinone of the formula 1

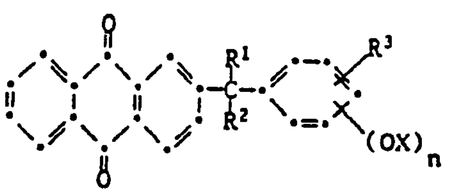

(I)

in which $R^1$ and $R^2$ independently of one another are H or $C_1$-$C_5$alkyl, $R^3$ is H, $C_1$-$C_5$alkyl, $-CH_2C_6H_4OH$ or $-C(CH_3)_2C_6H_4OH$, n is 1, 2 or 3 and X is H,

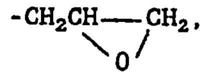

$-C_mH_{2m}COOH$ or $-C_mH_{2m}CONR'NH_2$, m is a number from 1 to 12 and R' is H or $C_1$-$C_6$alkyl.

2. An anthraquinone of the formula I according to claim 1, in which alkyl $R^1$ and $R^2$ independently of one another are methyl or ethyl, or $R^1$ and $R^2$ are H

3. An anthraquinone of the formula I according to claim 1, in which $R^3$ is H or methyl.

4. An anthraquinone of the formula I according to claim 1, in which $R^3$ is H, X is H or

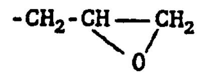

and n is 1, 2 or 3.

5. An anthraquinone of the formula I according to claim 1, in which $R^3$ is methyl, X is H or

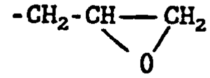

and n is 1

6. An anthraquinone of the formula I according to claim 1, in which $R^3$ is H, n is 1 or 2 and the group OX is bonded in the o- and/or p-position relative to the $-CR^1R^2$ group

7. An anthraquinone of the formula I according to claim 1, in which $R^3$ is $C_1$-$C_6$alkyl, n is 1, $R^3$ is in the o-position relative to the $-CR^1R^2$ group and $-OX$ is bonded in the p-position relative to $R^3$, or $-OX$ is bonded in the o-position relative to the $-CR^1R^2$ group and $R^3$ is bonded in the p-position relative to $-OX$.

8. An anthraquinone of the formula I according to claim 1, this being 2-[(2'-hydroxyphen-1'-yl)methyl]anthraquinone, 2-[(4'-hydroxyphen-1'yl)-methyl]anthraquinone, 2-[(2',4'-dihydroxyphen-1'-yl)methyl]-anthraquinone, 2-[(2',4',6'-trihydroxyphenyl)methyl]anthraquinone, 2-[(2'-hydroxy-5-methyl-phen-1'-yl)methyl]anthraquinone, 2-[(2'-methyl-5'-hydroxyphen-1'-yl)methyl]anthraquinone or a glycidyl derivative thereof.

9. A process for the preparation of an anthraquinone of the formula I according to claim 1, which comprises reacting a 2-(halogenomethyl)-anthraquinone of the formula II

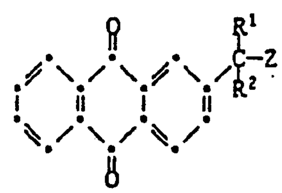

(II)

in which $R^1$ and $R^2$ are as described above and Z is halogen, with a phenol of the formula III

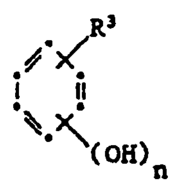

(III)

in which $R^3$ and n are as defined above, and, to prepare a compound of the formula I in which X is

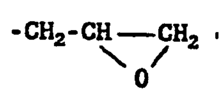

or $-C_mH_{2m}COOH$, reacting the product with epichlorohydrin or $Z'$-$C_mH_{2m}COOR$, in which R is the radical of an alcohol, in the presence of an HCl-binding agent, hydrolysing the ester and reacting the product with $NHR'NH_2$.

10. The process according to claim 9, wherein the reaction is carried out without a Lewis catalyst if n in formula III is 2 or 3.

11. The process according to claim 10, wherein the reaction is carried out in a polar aprotic solvent.

12. A curable composition containing

a) at least one epoxy resin with on average more than one epoxide group in the molecule,

b) if appropriate a curing agent for the epoxy resin,

c) at least one anthraquinone of the formula I according to claim 1 and

d) at least one primary or secondary aliphatic amine which contains at least one hydroxyl group in the aliphatic radical.

13. A composition according to claim 12, which contains

c) the anthraquinone of the formula I in an amount of 0.1-1 mol/kg of epoxy resin and

d) the amine in an amount of 0.1-1.2 mol/kg of epoxy resin.

14. A composition according to claim 12, which contains a glycidylated novolak, hydantoin, aminophenol, bisphenol or aromatic diamine as the epoxy resin.

15. A composition according to claim 14, in which the epoxy resin is an epoxidized cresol-novolak, bisphenol A diglycidyl ether, bisphenol A diglycidyl ether prelengthened with bisphenol A, 5,5-dimethylhydantoin N,N'-bisglycide, 1,3-bis(N'N'-diglycidyl-5,5-dimethylhydanto-4-yl)-2-glycidoxypropane, p-aminophenol triglycide, diaminodiphenylmethane or a mixture thereof

16. A composition according to claim 12, in which the curing agent b) is a novolak, a polyaminoamide or a polycarboxylic anhydride.

17. A composition according to claim 12, in which the amine of component d) has the formula IV

$$H\text{-}\underset{}{\overset{R^4}{\overset{|}{N}}}\text{-}C_yH_{2y+1-x}(OH)_x \qquad (IV)$$

in which $R^4$ is H, linear or branched $C_1$-$C_{18}$alkyl, $C_3$-$C_7$cycloalkyl, $C_6$-$C_{10}$aryl, $C_7$-$C_{18}$alkaryl, $C_7$-$C_{12}$aralkyl, $C_8$-$C_{18}$alkaralkyl or the group $-C_yH_{2y+1-x}(OH)_x$, x is a number from 1 to 3 and y is a number from 2 to 12, it being possible for the group $C_yH_{2y}$ to be interrupted by one or more $-O-$ or $-NR^4-$.

18. A composition according to claim 12, in which, in formula IV, $R^4$ is H, y is a number from 2 to 7 and x is a number from 1 to 3.

19. A cured composition of

a) at least one epoxy resin with on average more than one epoxide group in the molecule,

b) if appropriate a curing agent for the epoxy resin,

c) at least one anthraquinone of the formula I according to claim 1 and

d) at least one primary or secondary aliphatic amine which contains at least one hydroxyl group in the aliphatic radical.

20. The use of a cured composition according to claim 19 for the production of metallic coatings or images by currentless deposition of metal after complete or partial exposure of the surface to light.

## Revendications

1. Anthraquinones répondant à la formule I :

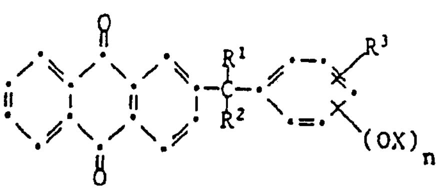

(I)

dans laquelle $R^1$ et $R^2$ représentent chacun, indépendamment l'un de l'autre, l'hydrogène ou un alkyle en $C_1$-$C_5$, $R^3$ représente l'hydrogène, un alkyle en $C_1$-$C_5$ ou un radical $-CH_2C_6H_4OH$ ou $-C(CH_3)_2C_6H_4OH$, ndésigne un nombre égal à 1, à 2 ou à 3 et X représente un radical

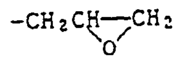

$-C_mH_{2m}$ COOH ou $-C_mH_{2m}$ CONR'$NH_2$, l'indice m désignant un nombre de 1 à 12 et R' représentant l'hydrogène ou un alkyle en $C_1$-$C_6$.

2. Anthraquinones de formule I selon la revendication 1 dans lesquelles $R^1$ et $R^2$, en tant qu'alkyles, représentent chacun, indépendamment l'un de l'autre, un méthyle ou un éthyle, ou $R^1$ et $R^2$ représentent chacun l'hydrogène.

3. Anthraquinones de formule I selon la revendication 1 dans lesquelles $R^3$ représente H ou un méthyle.

4. Anthraquinones de formule I selon la revendication 1 dans lesquelles $R^3$ représente H, X représente H ou

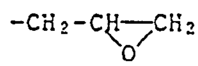

et n est égal à 1, à 2 ou à 3

5. Anthraquinones de formule I selon la revendication 1 dans lesquelles $R^3$ représente un méthyle, X représente H ou

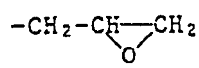

et n est égal à 1

6. Anthraquines de formule I selon la revendication 1 dans lesquelles $R^3$ représente H, n est égal à 1 ou à 2 et le radical OX se trouve en position ortho et/ou para relativement au radical $-CR^1R^2-$.

7. Anthraquinones de formule I selon la revendication 1 dans lesquelles $R^3$ représente un alkyle en $C_1$-$C_5$, n est égal à 1, $R^3$ est en position ortho relativement au radical $-CR^1R^2-$ et $-OX$ en position para relativement à $R^3$, ou $-OX$ est en position ortho par rapport au radical $-CR^1R^2-$ et $R^3$ en position para relativement à $-OX$.

8. Anthraquinones de formule I selon la revendication 1, en l'espèce :

1'[(hydroxy-2 phényl)-méthyl]-2 anthraquinone,
1'[(hydroxy-4 phényl)-méthyl]-2 anthraquinone,
1a [(dihydroxy-2,4 phényl)-méthyl]-2 anthraquinone,
1a [(trihydroxy-2,4,6 phényl)-méthyl]-2 anthraquinone,
1'[(hydroxy-2 méthyl-5 phényl)-méthyl]-2 anthraquinone,
1a [(méthyl-2 hydroxy-5 phényl)-méthyl]-2 anthraquinone,
ainsi que leurs dérivés glycidyliques

9. Procédé pour préparer des anthraquinones de formule I selon la revendication 1, procédé caractérisé en ce qu'on fait réagir une (halogénométhyl)-2 anthraquinone répondant à la formule II :

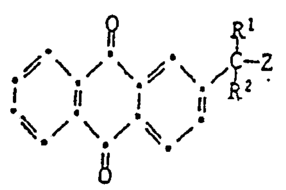

(II),

dans laquelle $R^1$ et $R^2$ ont les significations qui leur ont été données ci-dessus et Z représente un halogène, avec un phénol répondant à la formule III

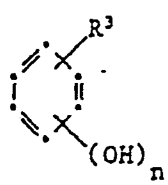

(III),

dans laquelle $R^3$ et n ont les significations qui leur ont été données ci-dessus, et, pour préparer des composés

de formule I dans lesquels X représente

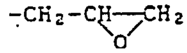

ou $-C_mH_{2m}$ COOH, on fait réagir, en présence d'un accepteur d'HCl, avec l'épichlorhydrine ou avec un composé Z'-$C_mH_{2m}$ COOR dans lequel R représente le radical d'un alcool, et on hydrolyse l'ester ou on le fait réagir avec NHR'-NH2.

10. Procédé selon la revendication 9 caractérisé en ce que la réaction est effectuée sans catalyseur de Lewis lorsque, dans la formule III, n est égal à 2 ou à 3.

11. Procédé selon la revendication 10 caractérisé en ce qu'il est effectué dans un solvant aprotique polaire.

12. Composition durcissable qui contient :

a) au moins une résine époxydique contenant en moyenne plus d'un radical époxy dans sa molécule,

b) éventuellement un durcisseur pour la résine époxydique,

c) au moins une anthraquinone de formule I selon la revendication 1 et

d) au moins une amine aliphatique primaire ou secondaire qui contient, dans le radical aliphatique, au moins un radical hydroxy.

13. Composition selon la revendication 12 dans laquelle :

c) l'anthraquinone de formule I est contenue en une quantité de 0,1 à 1 mol par kilogramme de la résine époxydique et

d) l'amine est contenue en une quantité de 0,1 à 1,2 mol par kilogramme de la résine époxydique.

14. Composition selon la revendication 12 dans laquelle il y a, comme résines époxydiques, des novolaques glycidylées, des hydantoïnes glycidylées, des aminophénols glycidylés, des bisphénols glycidylés, ou des diamines aromatiques glycidylées.

15. Composition selon la revendication 14 dans laquelle la résine époxydique est une novolaque crésolique époxydée, l'éther diglycidylique du bis-phénol A, un éther diglycidylique du bis-phénol A pré-allongé par du bisphénol A, la N,N'-diglycidyl diméthyl-5,5 hydantoïne, le glycidyloxy-2 bis-(N-glycidyl diméthyl-5,5 hydantoïnyl-1)-1,3 propane, le triglycide de l'amino-4 phénol, le diaminodiphénylméthane ou un mélange de ces composés.

16. Composition selon la revendication 12 dans laquelle le durcisseur b) est une novolaque, un polyaminoamide ou un anhydride d'acide polycarboxylique.

17. Composition selon la revendication 12 dans laquelle l'amine de la composante d) répond à la formule IV :

$$H-\overset{R^4}{\underset{}{N}}-C_yH_{2y+1-x}(OH)_x \qquad (IV)$$

dans laquelle $R^4$ représente l'hydrogène, un alkyle en $C_1$-$C_{18}$ linéaire ou ramifié, un cycloalkyle en $C_3$-$C_7$ un aryle en $C_6$-$C_{10}$, un alkylaryle en $C_7$-$C_{18}$, un aralkyle en $C_7$-$C_{17}$, un alkylaralkyle en $C_8$-$C_{18}$ ou un radical x désigne un nombre de 1 à 3 et y un nombre de 2 à 12, le radical $C_yH_{2y}$ pouvant être interrompu par un ou plusieurs radicaux –O– ou $-NR^4-$.

18. Composition selon la revendication 17 caractérisée en ce que, dans la formule IV, $R^4$ représente H, y désigne un nombre de 2 à 7 et x désigne un nombre de 1 à 3.

19. Composition durcie constituée

a)d'au moins une résine époxydique contenant en moyenne plus d'un radical époxy dans sa molécule,

b) éventuellement d'un durcisseur pour la résine époxydique,

c) d'au moins une anthraquinone de formule I selon la revendication 1 et

d) d'au moins une amine aliphatique primaire ou secondaire qui contient, sans le radical aliphatique, au moins un radical hydroxy.

20. Application d'une composition durcie selon la revendication 19 à la production de revêtements ou d'images métalliques par métallisation sans courant après une irradiation complète ou partielle de la surface.